# FASCICULE DE BREVET EUROPEEN

(11) **EP 1 137 377 B1**
(45) Date de publication et mention de la délivrance du brevet: **15.11.2006**
(21) Numéro de dépôt: 99958287.7
(22) Date de dépôt: 09.12.1999
(51) Int. Cl.: A61F 2/44

(54) **PROTHESE DE DISQUE INTERVERTEBRAL A PLOTS**
BANDSCHEIBENPROTHESE MIT ANSCHLÄGEN
INTERVETEBRAL DISC PROSTHESIS WITH CONTACT BLOCKS

(30) Priorité: 11.12.1998 FR 9815674; 22.07.1999 FR 9909522
(43) Date de publication de la demande: 04.10.2001
(73) Titulaire: Stryker Spine, 33610 Cestas (FR)
(72) Inventeur: GAUCHET, Fabien, F-60800 Duvy (FR); SAINT-MARTIN, P., Rés."les Jardins de Bellegrave, 85, avenue Roger Cohé 33600 Pessac (FR); KELLY, William, Montville, NJ 07045 (US)
(74) Mandataire: Le Forestier, Eric
(86) Numéro de dépôt international: PCT/FR1999/003075
(87) Numéro de publication internationale: WO 2000/035387

(56) Documents cités:
- EP-A- 0 317 972
- EP-A- 0 610 837
- EP-A- 0 642 775

## Description

L'invention concerne les prothèses de disque intervertébral.

On connaît, par exemple du document EP-0 356 112, de telles prothèses, comprenant deux plateaux et un corps en matériau compressible interposé entre les plateaux et fixé à ceux-ci. La prothèse vient remplacer le disque naturel après ablation de celui-ci, les plateaux étant en appui contre les plateaux vertébraux des vertèbres adjacentes. De telles prothèses permettent de reproduire dans une large mesure le comportement mécanique d'un disque naturel sain, notamment en compression ou en torsion autour d'un axe quelconque perpendiculaire à la direction longitudinale du rachis. Toutefois, elles ne donnent pas satisfaction pour deux autres mouvements : la rotation relative des deux plateaux autour d'un axe principal de la prothèse, c'est-à-dire autour de l'axe du rachis, et le déplacement relatif des deux plateaux en cisaillement, c'est-à-dire à coulissement dans un plan perpendiculaire à cet axe. Les prothèses connues offrent pour ces deux derniers mouvements une réaction mécanique insuffisante ou bien sont trop rigides pour les premiers mouvements.

Le document FR-A 2723841 divulgue une prothèse conforme au préambule de la revendication 1.

Un but de l'invention est de fournir une prothèse de disque intervertébral permettant d'approcher encore davantage le comportement d'un disque intervertébral naturel sain.

En vue de la réalisation de ce but, on prévoit selon l'invention une prothèse selon la revendication 1.

Ainsi, le plot offre une résistance mécanique lorsque la prothèse subit une contrainte de rotation autour de son axe principal ou une contrainte en cisaillement dans une direction perpendiculaire à cet axe. En outre, cette résistance est variable en fonction de la position relative des plateaux, par exemple s'ils sont plus ou moins rapprochés l'un de l'autre et/ou plus ou moins inclinés l'un par rapport à l'autre. En effet, la résistance au cisaillement et à la rotation précitée sera d'autant plus importante que le plot sera proche du plateau opposé et donc que le corps sera comprimé suivant l'axe. De plus, en fonction de la position du plot, une inclinaison relative des plateaux pourra rapprocher le plot du plateau opposé et donc accroître la résistance de la prothèse localement au voisinage du plot, ou au contraire éloigner le plot du plateau opposé, et ainsi réduire cette résistance. La prothèse a donc un comportement mécanique variable en fonction de la position relative des plateaux, ce qui la rapproche d'un disque naturel sain. Le ou chaque plot peut constituer en outre, si ses dimensions sont suffisamment importantes, une butée limitant l'un des mouvements relatifs de flexion ou de translation des plateaux. Bien entendu, ce qui vient d'être expliqué pour un plot est valable a fortiori lorsque la prothèse comprend plusieurs plots.

Un mode de réalisation est énoncé à la revendication 2.

Avantageusement, le plot est décentré par rapport au plateau qui le porte.

Ainsi, le comportement en résistance de la prothèse dépend fortement de l'axe de la flexion et du sens de cette flexion.

Avantageusement, le plot s'étend à distance du plateau autre que celui qui le porte lorsque la prothèse n'est pas sollicitée.

Avantageusement, le plot a une longueur comprise entre 0,60 d et 0,90 d où d est une distance séparant les deux plateaux lorsque la prothèse n'est pas sollicitée.

Avantageusement, pour le ou chaque plateau muni d'au moins un plot, le corps est immobilisé par rapport au plateau à l'égard d'un déplacement parallèlement au plateau seulement grâce au plot.

Ainsi, le corps est en appui sans ancrage sur le ou chaque plateau comportant un plot. Le montage a donc lieu simplement en empilant les plateaux et le corps. Le corps est notamment apte à être séparé du plateau sous l'effet d'une traction entraînant un déplacement en sens opposé au plateau, ce qui bien sûr n'est pas envisageable dans les conditions habituelles d'utilisation de la prothèse.

Avantageusement, le plot s'étend dans un logement du corps débouchant dans une face latérale du corps.

Avantageusement, le plot a une forme aplatie dans un plan radial à l'axe principal de la prothèse.

Ainsi, lors d'un cisaillement ou d'une torsion autour de l'axe principal de la prothèse, la surface d'appui entre le plot et le corps est importante, conduisant à une bonne répartition de la charge, bien que le volume du plot puisse être relativement faible.

Avantageusement, le plot a une forme aplatie dans un plan tangent à une direction circonférentielle à l'axe principal de la prothèse.

Avantageusement, le plot présente une face latérale cylindrique et le corps présente une face cylindrique en appui sur la face cylindrique du plot.

Avantageusement, le plot présente une face latérale s'étendant à l'extérieur du corps.

Ainsi, le plot empiète modérément sur le volume du corps.

Avantageusement, la face latérale du plot s'étend dans le prolongement d'une face latérale externe du corps.

Avantageusement, pour le ou chaque plot, le plateau autre que celui portant le plot présente une zone évidée constituant la partie en regard du plot lorsque la prothèse est au repos.

Ainsi, en cas de flexion ou de rapprochement extrême des plateaux, le ou chaque plot ne vient pas en butée contre le plateau opposé de sorte que le corps déformable encaissant les efforts continue à imposer le comportement mécanique de la prothèse.

Avantageusement, le plateau comporte au moins deux plots disposés symétriquement autour d'un centre du plateau.

Avantageusement, chaque plateau comporte au moins un plot, les plots se recouvrant suivant une direction parallèle à l'axe principal de la prothèse lorsque la prothèse n'est pas sollicitée.

On accroît ainsi l'effet anti-cisaillement.

Avantageusement, les plots se recouvrent sur une longueur comprise entre 0,35 d et 0,65 d où d est une distance séparant les deux plateaux lorsque la prothèse n'est pas sollicitée.

Avantageusement, lorsque la prothèse n'est pas sollicitée, les plots se recouvrent sur une longueur comprise entre 0,45 h et 0,85 h où h est une hauteur des plots parallèlement à l'axe principal de la prothèse.

Avantageusement, chaque plateau comporte au moins deux plots, les plots étant disposés en alternance autour de l'axe principal de la prothèse.

D'autres caractéristiques et avantages de l'invention apparaîtront encore dans la description suivante de deux modes préférés de réalisation donnés à titre d'exemples non limitatifs. Aux dessins annexés :
- la figure 1 est une vue éclatée en perspective d'une prothèse selon un premier mode préféré de réalisation, sans son soufflet ;
- la figure 2 est une vue latérale de la prothèse de la figure 1 avec arrachement partiel au niveau du soufflet ;
- la figure 3 est une vue en perspective d'une variante de réalisation de la prothèse du premier mode ;
- les figures 4 et 5 sont des vues en perspective respectivement à l'état monté et éclaté, d'une prothèse selon un deuxième mode préféré de réalisation ; et
- les figures 6 et 7 sont des vues respectivement en perspective et en plan du corps de la prothèse de la figure 4.

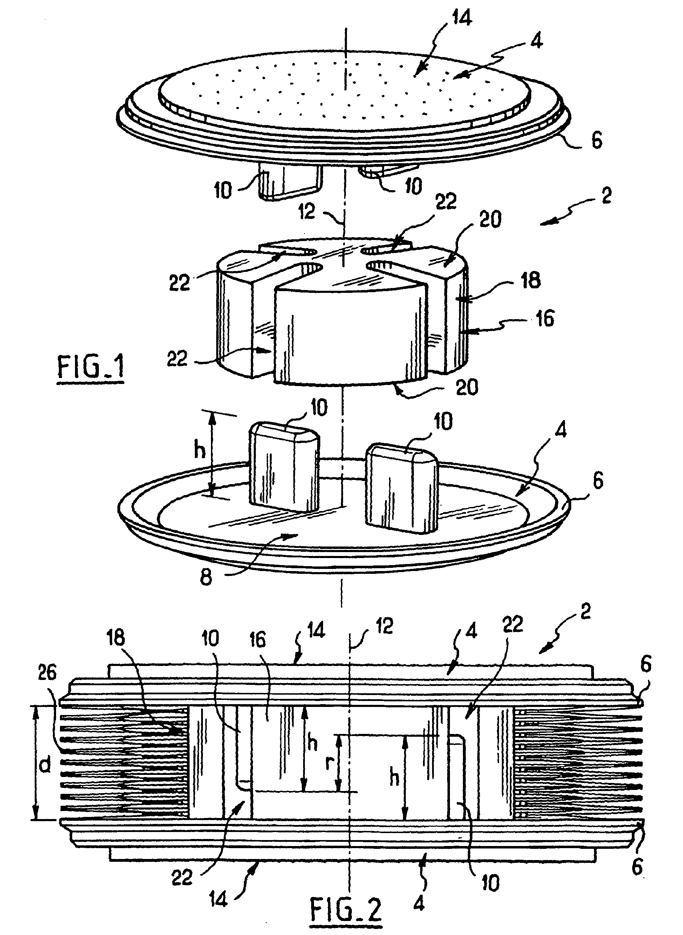

En référence aux figures 1 et 2, dans le présent mode de réalisation (ainsi que dans le deuxième mode), la prothèse de disque intervertébral 2 est destinée à une zone lombaire du rachis.

Elle comporte deux plateaux 4 identiques entre eux. Chaque plateau 4 a une forme générale de disque plan. En l'espèce, chaque plateau a un bord périphérique 6 légèrement relevé en saillie d'une face interne centrale plane 8 du disque, lui donnant ainsi une allure de soucoupe.

Chaque plateau comporte des plots 10, ici au nombre de deux, identiques entre eux. Chaque plot 10 a une forme générale de parallélépipède rectangle dont les arêtes, à l'exception de sa base en contact avec le plateau, auraient été arrondies. Le plot est sensiblement aussi haut que large mais est aplati suivant sa troisième dimension correspondant à son épaisseur. Les plots 10 s'étendent en saillie de la face interne 8, perpendiculairement au plan de celle-ci. Les deux plots 10 sont disposés symétriquement par rapport à un axe principal 12 de la prothèse passant par le centre de cette face et perpendiculaire à son plan lorsque la prothèse n'est pas sollicitée. L'épaisseur des plots 10 s'étend dans un plan radial à l'axe 12 de sorte que les deux plots sont sensiblement dans le même plan radial, de part et d'autre de l'axe 12. Sur une face externe plane 14, le plateau présente des reliefs facilitant la fixation du plateau 4 à un plateau vertébral notamment au moyen d'un revêtement d'hydroxyapatite. Les plateaux pourront être réalisés en métal ou en matériau composite.

La prothèse comporte un corps 16 de forme générale cylindrique, présentant une face externe cylindrique 18 et deux faces d'extrémité planes opposées 20. Le corps 16 est ici en un matériau élastique tel qu'un élastomère. Le corps présente des conduits ou logements 22, ici au nombre de quatre. Les conduits sont profilés suivant une direction parallèle à l'axe 12 du corps 16. Leur profil est identique au profil des plots 10 dans un plan transversal à leur hauteur, c'est-à-dire à l'axe 12. Chaque conduit 22 a donc une forme générale plane et s'étend dans un plan radial à l'axe 12. Chaque conduit 22 s'étend à distance de l'axe 12 et débouche sur la face cylindrique 18 sur toute sa hauteur ainsi que sur chacune des deux faces d'extrémité 20. Le corps a ainsi en vue d'extrémité une forme de croix ou de trèfle.

Pour le montage, le corps 16 est disposé entre les deux plateaux 4 ayant leurs faces internes 8 et leurs plots 10 en regard, les deux plateaux étant décalés relativement d'un quart de tour autour de l'axe 12, les quatre conduits 22 étant en regard des quatre plots. On rapproche les deux plateaux 4 l'un de l'autre jusqu'à mettre leurs faces internes 8 en contact surfacique avec les faces d'extrémité 20 du corps, les quatre plots 10 pénétrant dans les quatre conduits respectifs 22. Les quatre plots appartiennent ainsi aux deux plateaux en alternance autour de l'axe 12. Les plateaux 4 sont en appui sur le corps 16 sans autre ancrage que les plots. Le corps 16 est immobilisé par appui sur les faces internes 8 et les plots 10, ceux-ci interdisant à eux seuls son déplacement à translation dans un plan perpendiculaire à l'axe 12.

La prothèse comporte un soufflet compressible 26 en forme de manchon ayant un profil ondulé et fixé aux bords 6 des deux plateaux pour isoler de l'extérieur l'espace intérieur au soufflet, incluant le corps 16. En l'espèce, le soufflet présente dix convolutions, ce qui génère neuf crêtes en plus des crêtes fixées aux bords 6. Le soufflet ainsi que les plateaux peuvent être réalisés en titane ou alliage de titane.

Sur la figure 2, la prothèse est illustrée en l'absence de sollicitation. Chaque plot 10 a, suivant l'axe 12, une hauteur h comprise entre 0,60 d et 0,90 d où d est une distance, prise entre les bords 6, séparant les deux plateaux à l'état non sollicité de la prothèse. En l'espèce, h vaut 0,75 d.

Dans le présent mode, les plots 10 de l'un des plateaux sont en recouvrement partiel avec ceux de l'autre plateau dans la situation de la figure 2. Cela signifie que toute coupe de la prothèse transversale à l'axe 12 intercepte deux plots 10 d'un même plateau 4 au voisinage des faces d'extrémité 20, et les quatre plots dans une portion médiane du corps. La longueur de recouvrement r, mesurée parallèlement à l'axe 12, pourra être comprise entre 0,35 d et 0,65 d ou encore entre 0,45 h et 0,85 h. r vaut ici 0,66 h et 0,5 d.

Une fois installée, la prothèse se comporte comme suit.

Si la prothèse subit une rotation autour de l'axe 12, les plots 10 coopèrent avec le corps 16 pour encaisser une grande partie des contraintes générées, qui sont localement des contraintes de cisaillement.

Si la prothèse est comprimée suivant son axe 12, les quatre plots 10 pénètrent chacun davantage dans leur conduit 22, se déplaçant en direction du plateau opposé. La résistance de la prothèse au cisaillement perpendiculairement à l'axe 12 ou à la rotation autour de cet axe est donc plus importante.

Si la prothèse subit une flexion autour d'un axe perpendiculaire à l'axe 12, les deux plateaux 4 s'inclinent relativement, ce qui correspond localement à une compression sur certaines parties du corps 16 et à une traction sur d'autres parties du corps. La résistance au cisaillement est donc accrue dans les premières et réduite dans les secondes.

Bien que le corps 16 soit déformable, le mouvement de chaque plot 10 dans son conduit 22 s'apparente globalement à un coulissement.

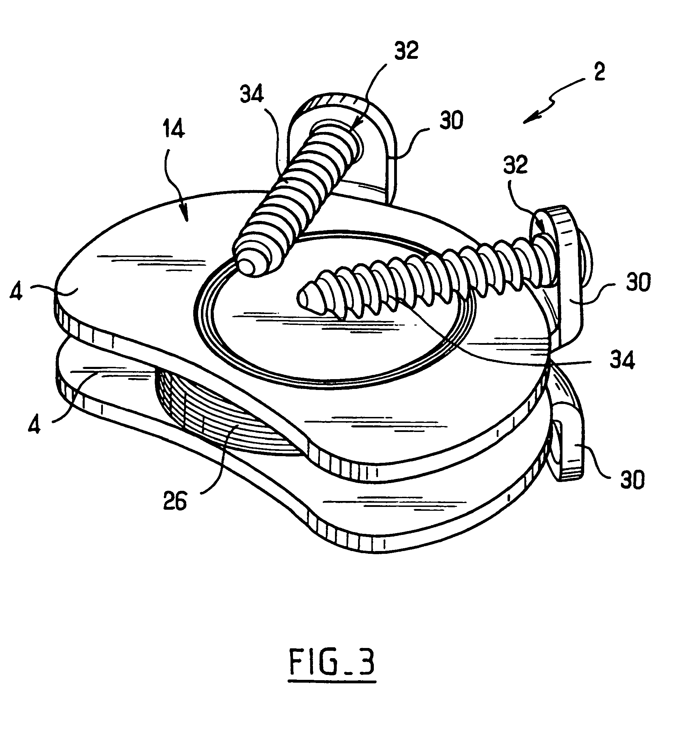

En référence à la figure 3, on pourra prévoir que chaque plateau 4 comporte deux pattes 30 s'étendant en saillie de la face externe 14 du plateau 4 perpendiculairement au plan du plateau. Chaque patte 30 présente un orifice 32 la traversant de part en part en direction du centre du plateau et, sur une face de la patte opposée au plateau, une empreinte de forme sphérique. Les orifices 32 permettent la réception d'une vis à os 34 ayant une tête dont une face inférieure a une forme sphérique mâle coopérant avec l'empreinte femelle de la patte 30 pour permettre une libre orientation de la vis par rapport à la patte associée.

Pour réaliser un ancrage à court terme de la prothèse de disque dans la colonne, on pourra ancrer les vis 34 dans le spondyle des vertèbres adjacentes au disque à remplacer.

Toutefois, on pourra prévoir un ancrage dit à long terme où, en outre, les surfaces 14 des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces pourront être traitées pour obtenir un état de surface plus ou moins poreux, présentant des points d'ancrage pour le tissu osseux, pour assurer une meilleure interface avec ledit tissu osseux. Sur la figure 3, les plateaux ont une forme de haricot à hile postérieur.

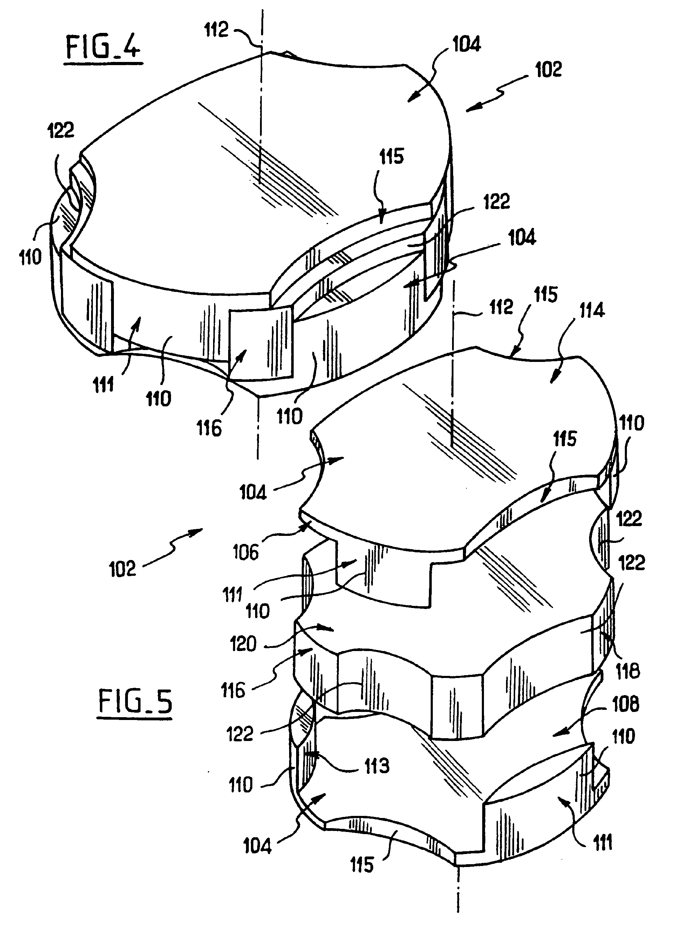

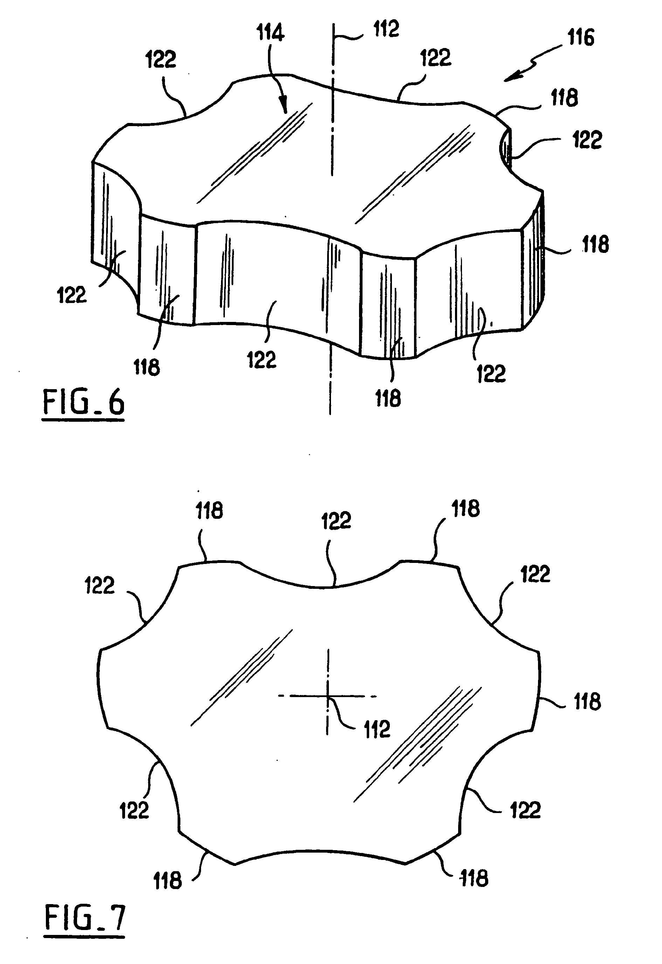

On a illustré aux figures 4 à 7 un deuxième mode de réalisation dans lequel les éléments analogues à ceux du premier mode portent des références numériques augmentées de 100.

Dans cette prothèse 102, chacun des deux plateaux 104 porte ici trois plots 110. Chaque plot 110 est contigu au bord 106 du plateau et a une forme plate parallèle à ce bord, c'est-à-dire sensiblement dans un plan tangent à une direction circonférentielle à l'axe 112. Le bord 106 ayant à l'endroit des plots 110 une forme en plan sensiblement circulaire convexe, chaque plot présente une face latérale externe 111 destinée à être opposée au corps 116 et de forme cylindrique convexe, s'étendant dans le prolongement du bord 106. Chaque plot 110 présente en outre une face latérale interne 113 également de forme cylindrique convexe. Les deux faces cylindriques 111, 113 de chaque plot forment deux arêtes parallèles à l'axe 112. Les arêtes sont en fait peu prononcées et très arrondies (dans un plan perpendiculaire à l'axe principal) pour éviter qu'elles n'entament le corps. Il en est de même pour les arêtes formant la face d'extrémité libre des plots. Ici encore, les plots des deux plateaux sont destinés à venir en recouvrement partiel parallèlement à l'axe 112 de sorte que les plots des deux plateaux s'imbriquent.

Le corps 116 présente une face latérale 118 de forme générale cylindrique, à ceci près que sa forme en plan est ici encore celle d'un haricot à hile postérieur. C'est également la forme en plan de la prothèse dans son ensemble. Les six conduits ou logements de plots 122 du corps 116 sont ici délimités par des faces cylindriques respectives concaves, d'axes parallèles à l'axe 112 et débouchant dans la face latérale 118. Le corps 116 présente donc latéralement une alternance de faces cylindriques concaves 122 et convexes 118. Chaque face 122 a le même rayon et la même longueur que la face latérale interne 113 du plot correspondant afin d'assurer un contact surfacique de l'une sur l'autre. Toutefois, la face cylindrique 122 du corps est plus haute, parallèlement à l'axe 112, que le plot.

Compte tenu de la forme en haricot de la prothèse, et puisque chaque plot est destiné à être reçu entre deux plots de l'autre plateau, les deux plateaux ne sont pas strictement identiques.

On assemble la prothèse comme précédemment en rapprochant mutuellement les deux plateaux 104 avec leurs plots 110 en regard, en interposant le corps 116 entre ceux-ci. Chaque plot 110 pénètre ainsi dans son logement 122, la face latérale interne 113 du plot venant en contact avec la face 122 du conduit. Une fois l'assemblage réalisé, comme sur la figure .4, chaque plateau a sa face plane interne en appui sur la face plane d'extrémité respective du corps.

Les courbures des faces latérales externes 111 des plots et de la face latérale 118 du corps (c'est-à-dire de chaque tronçon de cette face) sont choisies de sorte que ces faces s'étendent dans le prolongement les unes des autres sans arête saillante ou en creux, en donnant en plan à la prothèse une forme de haricot.

Chaque plateau 104 présente en plan trois évidements 115 débouchant dans le bord 106 et chacun délimité par un bord circulaire concave de plus grand rayon que celui de la face 122 associée comme on le verra. Chaque évidement 115 s'étend entre deux des plots 110 du plateau afin de s'étendre lui-même en regard d'un plot de l'autre plateau.

Ainsi, dans la position assemblée et au repos, comme sur la figure 4, chaque plot 110 s'étend en regard d'une partie de l'autre plateau constituée par l'évidement 115. Ainsi, en cas de forte sollicitation déplaçant au moins l'un des plots 110 en direction de l'autre plateau, le plot peut s'étendre jusque dans l'évidement 115 sans venir en butée contre le plateau. Le rayon ou la profondeur de l'évidement 115 est supérieur à celui du conduit 122 associé, ce qui donne à l'ensemble une configuration localement en escalier et prévient toute mise en butée du plot contre le plateau opposé. Cette grande profondeur de l'évidement accroît la flexibilité de la prothèse lors d'une torsion autour d'un axe perpendiculaire à l'axe principal:

Les caractéristiques concernant les dimensions r, h et d sont encore présentes dans ce mode de réalisation. Dans ce mode de réalisation également, on pourra prévoir un soufflet ou encore une fixation par vis comme sur la figure 3.

Ici encore, chaque plot 110 est mobile par rapport au corps et apte à solliciter le corps suivant une direction non parallèle à l'axe 112, cette sollicitation étant variable en fonction de la position du plot dans le corps suivant la direction radiale et la direction parallèle à l'axe 112. Bien que les plots pénètrent plus faiblement dans le corps que dans le précédent mode, ils assurent encore la transmission des contraintes des plateaux au corps.

La flexibilité en torsion de la prothèse peut être réglée notamment en modifiant l'épaisseur des trois plots 110. En effet, des plots plus épais rendent la prothèse plus rigide en torsion autour de l'axe 112. De plus, puisqu'il n'y a aucune fixation entre le corps 116 et chacun des plateaux 104, ceux-ci peuvent glisser dans une certaine mesure sur le corps durant la torsion. Le corps 116 disposé entre les plots 110 opposés se trouve comprimé et poussé radialement vers l'intérieur durant une torsion en raison du mouvement d'ensemble des plots.

La rigidité axiale de la prothèse peut être réglée notamment au niveau de la zone de contact entre le corps 116 et chaque plateau 104. En effet, on peut conformer la face interne de chaque plateau et la face d'extrémité associée du corps, de sorte que le contact entre ces faces s'établit sur une zone de plus en plus importante à mesure que croît la sollicitation en compression suivant l'axe 112. On pourra conserver par exemple la forme plane de la face d'extrémité du corps et donner à la face interne du plateau en regard une forme légèrement sphérique convexe. La rigidité axiale est notamment fonction du rayon de la face sphérique.

Afin d'assurer une fixation à long terme entre les plateaux et le corps, on pourra faire en sorte que le corps soit dans une large mesure emboîté dans les plateaux entre les plots de ceux-ci. Le fait que les plateaux ne sont pas rigidement fixés au corps facilite la fabrication ainsi que le réglage de la hauteur de la prothèse en préalable à l'intervention ou durant celle-ci. Il suffit en effet de modifier la hauteur du corps suivant l'axe 112, par exemple en changeant de corps, pour modifier la hauteur de la prothèse elle-même.

Lorsque la prothèse est soumise à une torsion autour de son axe principal, les zones du corps situées entre deux plots successifs sont comprimées, suivant une direction circonférentielle. Puisqu'il y a ici trois plots sur chaque plateau, il y a trois zones de compression. La rigidité en torsion dépend donc de la forme des plots, de la distance entre eux et de l'importance du recouvrement axial entre les plots.

Chacun des plateaux ainsi que le corps seront réalisés séparément par injection.

La prothèse supporte une mise en charge cyclique prolongée sans modification de sa forme.

Dans certaines prothèses connues, la liaison entre les plateaux et le corps est fragile et risque de rompre. La prothèse selon l'invention élimine ce risque puisque les plateaux sont prévus pour être mobiles en glissant sur le corps.

Les plots limitent l'extension du corps hors du contour des plateaux, notamment durant une compression axiale.

Le corps pourra avoir un module élastique variable dans l'espace.

Le corps pourra être en plusieurs matériaux.

Lorsqu'on utilise pour le corps un élastomère homogène, c'est-à-dire ayant partout même module d'élasticité, le ratio de la rigidité en compression axiale sur la rigidité en torsion est généralement trop élevé. Afin de réduire la rigidité axiale sans trop réduire la rigidité en torsion, on pourra mettre en oeuvre l'une des deux variantes suivantes. Dans une première variante, on peut réaliser le centre du corps dans un élastomère moins rigide que celui de la périphérie du corps. Cela réduit la rigidité en compression sans beaucoup affecter la rigidité en torsion puisque cette dernière est régie surtout par la périphérie du corps. Dans une deuxième variante, on peut ménager une cavité dans la surface interne des plateaux en contact avec le corps, afin de réduire la surface de contact des plateaux avec le corps. Cela réduit le volume d'élastomère qui se trouve comprimé en cas de compression axiale, diminuant ainsi la rigidité axiale, sans ici encore, beaucoup affecter la rigidité en torsion.

Bien entendu, on pourra apporter à l'invention de nombreuses modifications sans sortir du cadre de celle-ci.

Le plot 10, s'il est unique sur un plateau, pourra être au centre du plateau qui le porte.

Le ou chaque plot 10, 110 pourra être en contact avec le plateau opposé 4, 104 lorsque la prothèse n'est pas sollicitée ou bien seulement lorsque la compression de la prothèse au niveau du plot dépasse une certaine limite : le plot forme ainsi une butée limitant certains types de mouvements.

On pourra envisager que seul l'un des plateaux 4, 104 porte un ou plusieurs plots 10, 110.

Le corps 16, 116 pourra être réalisé dans un matériau viscoélastique tel que du silicone.

Les plots pourront avoir une autre forme, par exemple une forme cylindrique d'axe parallèle à l'axe 12, 112.

Le plot pourra s'étendre dans un logement du corps ne débouchant pas sur une face latérale du corps.

## Revendications

1. Prothèse de disque intervertébral (2 ; 102) comportant deux plateaux (4 ; 104) et un corps déformable (16 ; 116) interposé entre les plateaux, au moins l'un des plateaux (4 ; 104) comportant au moins un plot (10 ; 110) mobile par rapport au corps (16 ; 116) **caractérisée en ce que** le plot est apte à solliciter le corps suivant une direction non parallèle à un axe principal (12 ; 112) de la prothèse, passant perpendiculairement par les plateaux.

2. Prothèse selon la revendication 1, **caractérisée en ce que** le plot est en contact avec ledit corps (16 ; 116).

3. Prothèse selon la revendication 1 ou 2, **caractérisée en ce que** le plot (10 ; 110) est décentré par rapport au plateau (4 ; 104) qui le porte.

4. Prothèse selon l'une quelconque des revendications 1 à 3, **caractérisée en ce que** le plot (10 ; 110) s'étend à distance du plateau autre que celui qui le porte lorsque la prothèse n'est pas sollicitée.

5. Prothèse selon l'une quelconque des revendications 1 à 4, **caractérisée en ce que** le plot (10 ; 110) a une longueur (h) comprise entre 0,60 d et 0,90 d où d est une distance séparant les deux plateaux (4 ; 104) lorsque la prothèse n'est pas sollicitée.

6. Prothèse selon l'une quelconque des revendications 1 à 5, **caractérisée en ce que**, pour le ou chaque plateau muni d'au moins un plot (10 ; 110), le corps (16 ; 116) est immobilisé par rapport au plateau à l'égard d'un déplacement parallèlement au plateau seulement grâce au plot.

7. Prothèse selon l'une quelconque des revendications 1 à 6, **caractérisée en ce que** le plot (10 ; 110) s'étend dans un logement (22 ; 122) du corps (16 ; 116) débouchant dans une face latérale (18 ; 118) du corps.

8. Prothèse selon l'une quelconque des revendications 1 à 7, **caractérisée en ce que** le plot (10) a une forme aplatie dans un plan radial à l'axe principal (12) de la prothèse.

9. Prothèse selon l'une quelconque des revendications 1 à 7, **caractérisée en ce que** le plot (110) a une forme aplatie dans un plan tangent à une direction circonférentielle à l'axe principal (112) de la prothèse.

10. Prothèse selon l'une quelconque des revendications 1 à 9, **caractérisée en ce que** le plot (110) présente une face latérale cylindrique et le corps présente une face cylindrique en appui sur la face cylindrique du plot.

11. Prothèse selon l'une quelconque des revendications 1 à 10, **caractérisée en ce que** le plot (110) présente une face latérale (111) s'étendant à l'extérieur du corps (116).

12. Prothèse selon l'une quelconque des revendications 1 à 11, **caractérisée en ce que** la face latérale (111) du plot (110) s'étend dans le prolongement d'une face latérale externe (118) du corps (116).

13. Prothèse selon l'une quelconque des revendications 1 à 12, **caractérisée en ce que** pour le ou chaque plot (110), le plateau (104) autre que celui portant le plot présente une zone évidée (115) constituant la partie en regard du plot lorsque la prothèse est au repos.

14. Prothèse selon l'une quelconque des revendications 1 à 13, **caractérisée en ce que** le plateau (4 ; 104) comporte au moins deux plots (10 ; 110) disposés symétriquement autour d'un centre du plateau.

15. Prothèse selon l'une quelconque des revendications 1 à 14, **caractérisée en ce que** chaque plateau (4 ; 104) comporte au moins un plot (10 ; 110), les plots se recouvrant suivant une direction parallèle à l'axe principal (12 ; 112) de la prothèse lorsque la prothèse n'est pas sollicitée.

16. Prothèse selon la revendication 15, **caractérisée en ce que** les plots (10 ; 110) se recouvrent sur une longueur (r) comprise entre 0,35 d et 0,65 d où d est une distance séparant les deux plateaux (4 ; 104) lorsque la prothèse n'est pas sollicitée.

17. Prothèse selon la revendication 15 ou 16, **caractérisée en ce que**, lorsque la prothèse n'est pas sollicitée, les plots (10 ; 110) se recouvrent sur une longueur (r) comprise entre 0,45 h et 0,85 h où h est une hauteur des plots parallèlement à l'axe principal (12 ; 112) de la prothèse.

18. Prothèse selon l'une quelconque des revendications 1 à 17, **caractérisée en ce que** chaque plateau (4 ; 104) comporte au moins deux plots (10 ; 110), les plots étant disposés en alternance autour de l'axe principal (12 ; 112) de la prothèse.

## Claims

1. Intervertebral disc prosthesis (2; 102) comprising two plates (4; 104) and a deformable body (16; 116) interposed between the plates, at least one of the plates (4; 104) comprising at least one contact stud (10; 110) that is movable relative to the body (16; 116), **characterized in that** the contact stud is able to stress the body in a direction not parallel to a main axis (12; 112) of the prosthesis that passes perpendicularly through the plates.

2. Prosthesis according to Claim 1, **characterized in that** the contact stud is in contact with said body (16; 116).

3. Prosthesis according to Claim 1 or 2, **characterized in that** the contact stud (10; 110) is offcentred relative to the plate (4; 104) which bears it.

4. Prosthesis according to any one of Claims 1 to 3, **characterized in that** the contact stud (10; 110) extends at a distance from the plate other than the one which bears it when the prosthesis is not stressed.

5. Prosthesis according to any one of Claims 1 to 4, **characterized in that** the contact stud (10; 110) has a length (h) of between 0.60 d and 0.90 d, where d is a distance separating the two plates (4; 104) when the prosthesis is not stressed.

6. Prosthesis according to any one of Claims 1 to 5, **characterized in that**, for the plate or each plate provided with at least one contact stud (10; 110), the body (16; 116) is immobilized relative to the plate, in respect of a displacement parallel to the plate, only by virtue of the contact stud.

7. Prosthesis according to any one of Claims 1 to 6, **characterized in that** the contact stud (10; 110) extends in a recess (22; 122) of the body (16; 116) opening into a lateral face (18; 118) of the body.

8. Prosthesis according to any one of Claims 1 to 7, **characterized in that** the contact stud (10) has a flattened shape in a plane radial to the main axis (12) of the prosthesis.

9. Prosthesis according to any one of Claims 1 to 7, **characterized in that** the contact stud (110) has a flattened shape in a plane tangential to a direction circumferential to the main axis (112) of the prosthesis.

10. Prosthesis according to any one of Claims 1 to 9, **characterized in that** the contact stud (110) has a cylindrical lateral face, and the body has a cylindrical face bearing on the cylindrical face of the contact stud.

11. Prosthesis according to any one of Claims 1 to 10, **characterized in that** the contact stud (110) has a lateral face (111) extending outside the body (116).

12. Prosthesis according to any one of Claims 1 to 11, **characterized in that** the lateral face (111) of the contact stud (110) extends in the continuation of an outer lateral face (118) of the body (116).

13. Prosthesis according to any one of Claims 1 to 12, **characterized in that**, for the contact stud or each contact stud (110), the plate (104) other than the one bearing the contact stud has a recessed zone (115) constituting the part facing the contact stud when the prosthesis is at rest.

14. Prosthesis according to any one of Claims 1 to 13, **characterized in that** the plate (4; 104) comprises at least two contact studs (10; 110) arranged symmetrically about a centre of the plate.

15. Prosthesis according to any one of Claims 1 to 14, **characterized in that** each plate (4; 104) comprises at least one contact stud (10; 110), the contact studs overlapping in a direction parallel to the main axis (12; 112) of the prosthesis when the prosthesis is not stressed.

16. Prosthesis according to Claim 15, **characterized in that** the contact studs (10; 110) overlap by a length (r) of between 0.35 d and 0.65 d, where d is a distance separating the two plates (4; 104) when the prosthesis is not stressed.

17. Prosthesis according to Claim 15 or 16, **characterized in that** when the prosthesis is not stressed, the contact studs (10; 110) overlap by a length (r) of between 0.45 h and 0.85 h, where h is a height of the contact studs parallel to the main axis (12; 112) of the prosthesis.

18. Prosthesis according to any one of Claims 1 to 17, **characterized in that** each plate (4; 104) comprises at least two contact studs (10; 110), the contact studs being arranged alternatingly around the main axis (12; 112) of the prosthesis.

## Patentansprüche

1. Bandscheibenprothese (2; 102), mit zwei Platten (4; 104) und einem zwischen den Platten angeordneten deformierbaren Körper (16; 116), wobei wenigstens eine der Platten (4; 104) wenigstens ein bezüglich des Körpers (16; 116) bewegliches Kontaktstück (10; 110) umfaßt, **dadurch gekennzeichnet, daß** das Kontaktstück in der Lage ist, den Körper in einer Richtung nichtparallel zu,einer senkrecht durch die Platten laufenden Hauptachse (12; 112) der Prothese zu beanspruchen.

2. Prothese nach Anspruch 1, **dadurch gekennzeichnet, daß** das Kontaktstück in Kontakt mit dem Körper (16; 116) steht.

3. Prothese nach Anspruch 1 oder 2, **dadurch gekennzeichnet, daß** das Kontaktstück (10; 110) bezüglich der es tragenden Platte (4; 104) dezentriert ist.

4. Prothese nach einem der Ansprüche 1 bis 3, **dadurch gekennzeichnet, daß** sich das Kontaktstück (10; 110) mit Abstand zur anderen als es tragenden Platte erstreckt, wenn die Prothese nicht beansprucht ist.

5. Prothese nach einem der Ansprüche 1 bis 4, **dadurch gekennzeichnet, daß** das Kontaktstück (10; 110) eine Länge (h) zwischen 0,60 d und 0,90 d hat, wobei d ein Abstand zwischen den beiden Platten (4; 104) ist, wenn die Prothese nicht beansprucht ist.

6. Prothese nach einem der Ansprüche 1 bis 5, **dadurch gekennzeichnet, daß** für die oder jede mit wenigstens einem Kontaktstück (10; 110) ausgestattete Platte der Körper (16; 116) bezüglich der Platte gegenüber einer Verschiebung parallel zur Platte nur aufgrund des Kontaktstückes unbeweglich ist.

7. Prothese nach einem der Ansprüche 1 bis 6, **dadurch gekennzeichnet, daß** das Kontaktstück (10; 110) sich in eine Aufnahme (22; 122) des Körpers (16; 116) erstreckt, die in eine Seitenfläche (18; 118) des Körpers mündet.

8. Prothese nach einem der Ansprüche 1 bis 7, **dadurch gekennzeichnet, daß** das Kontaktstück (10) eine abgeflachte Form in einer Ebene radial zur Hauptachse (12) der Prothese hat.

9. Prothese nach einem der Ansprüche 1 bis 7, **dadurch gekennzeichnet, daß** das Kontaktstück (110) eine abgeflachte Form in einer Ebene tangential zu einer Umfangsrichtung zur Hauptachse (112) der Prothese hat.

10. Prothese nach einem der Ansprüche 1 bis 9, **dadurch gekennzeichnet, daß** das Kontaktstück (110) eine zylindrische Seitenfläche aufweist und der Körper eine an der zylindrischen Fläche des Kontaktstückes anliegende zylindrische Fläche aufweist.

11. Prothese nach einem der Ansprüche 1 bis 10, **dadurch gekennzeichnet, daß** das Kontaktstück (110) eine Seitenfläche (111) aufweist, die sich ins Innere des Körpers (116) erstreckt.

12. Prothese nach einem der Ansprüche 1 bis 11, **dadurch gekennzeichnet, daß** die Seitenfläche (111) des Kontaktstückes (110) sich in die Verlängerung einer Außenseitenfläche (118) des Körpers (116) erstreckt.

13. Prothese nach einem der Ansprüche 1 bis 12, **dadurch gekennzeichnet, daß** für das oder jedes Kontaktstück (110) die andere als das Kontaktstück tragende Platte (104) einen ausgesparten Bereich (115) aufweist, der den dem Kontaktstück gegenüberliegenden Abschnitt bildet, wenn die Prothese in Ruheposition ist.

14. Prothese nach einem der Ansprüche 1 bis 13, **dadurch gekennzeichnet, daß** die Platte (4; 104) wenigstens zwei Kontaktstücke (10; 110) umfaßt, die symmetrisch um ein Zentrum der Platte angeordnet sind.

15. Prothese nach einem der Ansprüche 1 bis 14, **dadurch gekennzeichnet, daß** jede Platte (4; 104) wenigstens ein Kontaktstück (10; 110) umfaßt, wobei die Kontaktstücke in einer Richtung parallel zur Hauptachse (12; 112) der Prothese verlaufen, wenn die Prothese nicht beansprucht ist.

16. Prothese nach Anspruch 15, **dadurch gekennzeichnet, daß** die Kontaktstücke (10; 110) über eine Länge (r) zwischen 0,35 d und 0,65 d verlaufen, wobei d ein Abstand zwischen den beiden Platten (4; 104) ist, wenn die Prothese nicht beansprucht ist.

17. Prothese nach Anspruch 15 oder 16, **dadurch gekennzeichnet, daß** die Kontaktstücke (10; 110), wenn die Prothese nicht beansprucht ist, über eine Länge (r) zwischen 0,45 h und 0,85 h verlaufen, wobei h eine Höhe der Kontaktstücke parallel zur Hauptachse (12; 112) der Prothese ist.

18. Prothese nach einem der Ansprüche 1 bis 17, **dadurch gekennzeichnet, daß** jede Platte (4; 104) wenigstens zwei Kontaktstücke (10; 110) umfaßt, wobei die Kontaktstücke abwechselnd um die Hauptachse (12; 112) der Prothese angeordnet sind.
